# EUROPEAN PATENT APPLICATION

(11) **EP 4 000 578 A1**
(43) Date of publication of application: **25.05.2022**
(21) Application number: 20917812.8
(22) Date of filing: 30.03.2020
(51) Int. Cl.: A61H 3/00, G16H 20/30, G16H 50/70

(54) **NEUROREHABILITATION SYSTEM AND METHOD FOR NEUROREHABILITATION**

(30) Priority: 07.02.2020 RU 2020105811
(71) Applicant: Vibraint Inc., Richmond Hill, Ontario L4E 3W4 (CA)
(72) Inventor: AVERKIEV, Maksim Konstantinovich, Samara, 443013 (RU); BORISHCHEV, Ilia Vladimirovich, Samara, 443110 (RU); BULANOV, Vladimir Aleksandrovich, Samara, 443031 (RU); KUCHKIN, Dmitriy Vladimirovich, Samara, 443051 (RU); MUKHIN, Oleg Aleksandrovich, Samara, 443115 (RU); POTANTSEV, Iurii Anatolievich, Samara, 443111 (RU)
(74) Representative: Osmans, Voldemars
(86) International application number: PCT/RU2020/000167
(87) International publication number: WO 2021/158138

(57) **Abstract**

The system of neurorehabilitation and the method of neurorehabilitation belong to the medical field, specifically to neurology, and can be used as a system and method of neurorehabilitation during motor rehabilitation of patients after a stroke in various phases, as well as during the rehabilitation of patients with other diseases and disorders of the central nervous system. The objective of the claimed invention is the motor (movement) rehabilitation of patients after stroke and other diseases of the central nervous system that cause motor deficits in the limbs. The technical result of the invention is to increase the effectiveness of rehabilitation, including during early and late rehabilitation period of stroke and other diseases and disorders of the central nervous system through the use of the system and method of neurorehabilitation and methods of this invention, which stimulate the restoration of mobility of paralyzed limbs by forming neural biofeedback connections between the patient's intention to move the limb and its implementation.

## Description

### Field of application

The invention belongs to the field of medicine, specifically to neurology and can be used as a system and method of neurorehabilitation in motor rehabilitation of patients after stroke during various stages, as well as in the rehabilitation of patients with other disorders of the central nervous system.

### State of the art

A functional biological control device RU2071723 is known from the prior art. The device includes physiological parameters sensors, a unit for converting these parameters into feedback signals and a unit for indicating these signals, and differs in that the unit for indicating feedback signals is made in the form of a generator of visual images that render a game-like situation.

The disadvantage of this device is that it is not effective in the rehabilitation of patients with post-stroke neurology symptomatics.

There is a known "Method of rehabilitation of patients in various stages of disorders of the central or peripheral nervous system using virtual reality" RU2655200. It uses a virtual environment with controls and sensory interaction with a virtual object. Taking into account the information obtained from the recording electroencephalographic and electromyographic sensors installed on the head and the affected limb, respectively, as well as the patient's ability to move, the volume of control of virtual movements is adjusted in such a way that a feeling of completion of the movement appears when virtual reality tasks are demonstrated. Sensory interaction with virtual objects through the use of the visual, auditory canal, as well as tactile and proprioceptive stimulation of the limb receptors is carried out in such a way as to ensure the patient's association with a virtual avatar, with a sense of tactile and proprioceptive contact with virtual objects and a sense of performing the movement. This method allows, through the use of virtual reality, the restoration of limb movement and walking functions in patients with damage caused to the central or peripheral nervous system, as well as with pathology of the musculoskeletal system, taking into account the information received from the recording electroencephalographic and electromyographic sensors.

The disadvantage of this method is insufficient effectiveness in the rehabilitation of patients in early rehabilitation of stroke, because it does not work with the visual evoked potential signal.

Also, there is a known "Method of rehabilitation of post-stroke and post-traumatic patients" RU2622206. The patient is presented with a task of the kinesthetic imagination of limb movement, and the patterns of the patient's brain activity created by the imaginary movement are analyzed. The data is transferred to a computer to extract signals related to the intended movement. Based on visual feedback, the patient is presented with the results of recognition of the task being performed in the form of a visual label on the screen. By changing the label, the correctness of the task execution is determined. The results of recognition of the performed task by the kinesthetic imagination of the movement of the paretic limb are additionally presented by tactile and proprioceptive feedback by means of an exoskeleton worn on the patient's paretic limb. With correct recognition of the task being performed, the exoskeleton moves the limb in the direction of the imaginary movement, and if the result is incorrect, in the opposite direction. The method allows to increase the effectiveness of treatment, which is achieved due to the additional involvement of tactile proprioceptive sensitivity in the restoration of motor functions.

The disadvantage of this method is its insufficient effectiveness in the rehabilitation of patients in early rehabilitation of stroke, because it does not work with the visual evoked potential signal.

Also, there is a known EP0911015 "Orthopedic rehabilitation apparatus using virtual reality devices". The device contains: an exoskeleton adapted to support the user's body, connected at its joints and equipped with small actuators ("microcylinders"), powered by compressed air, hydraulically or electrically, designed to move the jointed parts of the exoskeleton in accordance with a person's gait; a programmed control unit for controlling the operation of said actuators for moving the exoskeleton in accordance with a person's gait; a remote control unit for controlling a programmed control unit with commands to stop, start, or control the speed of a person's gait; an electronic virtual reality unit for transmitting to the user pictures of virtual reality and stimulation interactive with the patient's gait via a virtual reality headset; a rail suspended at a distance from the ground higher than a person's height to support and guide a bearing sliding on the rail; a metal frame supported by said bearing or slider and provided with two suspensions or rods to support the patient's exoskeleton.

The disadvantage of such a device is low efficiency for rehabilitation of patients with post-stroke neurosympatomatics due to the fact that the patient's intentions to make a movement are not being recorded and taken into account.

There is a known training system for the rehabilitation of the upper limbs CN109568083 (publ. 04/05/2019). The invention discloses a multimodal interactive system of robotic training for upper limb rehabilitation. The system contains a module for collecting and processing electroencephalogram (EEG), a robotic module, a complex module for collecting and processing data on the muscles of the affected limbs, a training rehabilitation assessment module and a virtual reality module, in which the EEG signal collection and processing module reflects the patient's intention to move; a robotic module helps an affected limb to perform rehabilitation exercises; a comprehensive module for collecting and processing data on the affected limb receives complex data indicators for the affected limb; the training rehabilitation evaluation module is used to process and analyze the complex data of the muscle indices of the affected limb in order to obtain quantitative parameters for evaluating improvements after the rehabilitation training of the patient's arms; the virtual reality module is used to display a virtual rehabilitation teaching environment and interacts with the patient through virtual scene display and dialogue.

The disadvantage of such a system is the inability to work with signals of visual evoked potential, because it works only with the signals of the movement intention, which leads to a decrease in the effectiveness of rehabilitation, especially in early rehabilitation of stroke and in cases where there is a high degree of paralysis of the limb. This rehabilitation training system works with EEG signals of brain activity and does not have the ability to use signals from functional near infrared spectroscopy. It is not possible to register signals of brain activity using magnetic resonance imaging and magnetic fields created by the electrical activity of the brain. Also, it does not register muscle activity and electrical stimulation. There is no possibility of activating mirror neurons, as well as the ability to display the instant feedback on the degree of completion of the assigned task based on registered signals of brain activity. In addition, the training system for upper limb rehabilitation is aimed at rehabilitation and training of the upper limbs only and cannot be used in the rehabilitation of the lower limbs.

Also, there is a known method of rehabilitation of the upper limbs CN106621287 (publ. 05/10/2017). The invention discloses a method for upper limb rehabilitation based on a brain-computer interface and virtual reality technology. An EEG cap and VR headset are put on the patient. A computer, EEG amplifier and smartphone are connected into a single system. During training, the VR headset and a smartphone create a first-person upper-limb training scene. The patient controls the movements of the upper limb in the virtual scene in real time mode, and the BCI module in the computer automatically adjusts the classifier in accordance with the patient's current training efficiency; after completing a training session, the BCI module in the computer automatically adjusts the classifier according to the patient's current training effect.

The disadvantages of this method are that it does not allow working with the signals of the visual evoked potential and only works with the signal of the motor imagination, which leads to a decrease in the effectiveness of rehabilitation, especially in early stroke and in cases where there is a high degree of paralysis of the limb. Also, this method is aimed at working with an electrical signal of brain activity and does not use signals from functional near infrared spectroscopy; it is not possible to register brain activity signals using magnetic resonance imaging and magnetic fields arising from electrical activity of the brain. There is no possibility of activating mirror neurons, nor is it able to display the degree of completion of the assigned task in instant feedback mode on the basis of registered signals of brain activity. Electromyostimulation and electromyography have not been implemented. In addition, this method is aimed at rehabilitation and training only of the upper limbs and cannot be used in the rehabilitation of the lower limbs.

There is a known exerciser for restoring mobility of fingers RU147759 (publ. 20.11.2014). The utility model relates to medicine, intended for the rehabilitation of patients with paralysis of the upper limbs and is aimed at providing the ability to move each finger of the hand according to the patient's mental commands. This result is achieved by the fact that the exerciser for restoring the mobility of the fingers contains an exoskeleton of the hand, actuators for moving the fingers of the exoskeleton with a control unit; at the same time, it is equipped with an individual actuators for moving each of the fingers, equipped with a means of attracting the patient's attention, and the input of the control unit for the actuators of the fingers is connected to an electroencephalographic cap worn on the patient's head, while the control unit contains a serially connected unit for recording an electroencephalogram, an analysis unit for an electroencephalogram and a unit for formation of commands to the actuators of the fingers.

The disadvantage of this exerciser is the use of a light-emitting diode as a visual display device to attract the patient's attention, and as a result, insufficient immersion of the patient in the training process and, as a consequence, a decrease in the effectiveness of the system. The disadvantages of this exerciser can also be attributed to the fact that it is intended for training and rehabilitation only of the part of the upper limb, namely the hand, and is not suitable for training and rehabilitation of the arm as a whole and of the lower limbs. In addition, there is no possibility of activating mirror neurons, nor is it able to display the degree of completion of the assigned task in instant feedback mode, based on the registered signals of brain activity. It is not possible to register signals of brain activity using magnetic resonance imaging and magnetic fields caused by electrical activity of the brain.

### Disclosure of terms

**A computer** is a device or system capable of performing a given, well-defined, variable sequence of operations, as well as any device or group of interconnected or adjacent devices, one or more of which, acting in accordance with the program, performs automated data processing. Within the framework of the description below, a computer can be represented in the form and be located in a personal computer (which is the most preferable option), in a mobile device (phone, smartphone, etc.). It is also possible to locate it remotely (for example, on a server, on a local network device or in the cloud), or to locate it on a microcomputer or several microcomputers built into one or more of the system elements.

**Commands** based on the interpretation of recorded signals of brain activity are commands that are received on the basis of computer-processed signals of brain activity for transmission to a robotic device. For example, it can be a command sent to an exoskeleton of a limb, to impact a trained object - for example, to move a limb or perform a certain movement. It can be also other physical impact on the trained object using a robotic device. The command also can be a command transmitted to the visual display device to demonstrate to the rehabilitated patient the process or degree of task completion.

**The trained object** is usually a limb (arm, leg), as well as parts of the limbs, for example, the foot, knee, ankle, shoulder, forearm or hand, fingers and other parts of the body affected by paralysis or paresis, that therefore require motor (movement) rehabilitation. There is also an **untrained object** - a healthy limb or its part symmetrical to the one being rehabilitated relative to the longitudinal axis of the human body.

**A database** is a systematized collection of information necessary for the operation of the system, including a set of reference signals ( specific patterns) of brain activity that are caused by the execution of a mental task, including those in response to commands and stimuli received during the task. The database is filled with reference signals (patterns) based on the results of the classifiers training.

**A classifier** is a software and its algorithm that, after training, identifies patterns of brain activity that are caused by performing a task.

**Neuroplasticity** is a process in the brain that restores neural connections to replace those lost or damaged as a result of a disease.

**Registration of brain activity** is the process of registering signals that occur in the brain as a result of the electrochemical activity of neurons. During a human's thinking or experiencing a variety of emotions and feelings, neurons interact with each other through special processes (nerve fibers) called axons. This kind of interaction has an electrochemical nature. When large groups of neurons (hundreds of thousands) interact at the same time, as a result of electrochemical activity, an electric field is generated with sufficient power to be recorded from outside of the head.

### BRIEF DESCRIPTION OF INVENTION

The object of the claimed invention is motor (movement) rehabilitation of patients after stroke and other diseases of the central nervous system that cause motor deficits in the limbs.

The technical result of the invention is increasing the effectiveness of rehabilitation, including in early and chronic stroke and other diseases (disorders) of the central nervous system through the use of the system and practice of neurorehabilitation and methods of this invention, which stimulate the restoration of mobility of paralyzed limbs by forming neural biofeedback connections between the patient's intention to move the limb and its implementation.

The neurorehabilitation system of this invention includes:
a visual display device, a device for registering brain activity, a robotic device for impacting a trained object, a computer, a database, as well as software for recognizing and extracting a recorded signal of brain activity and interpreting the registered and extracted signal using the database. The computer with the software is capable of transmitting commands formed on the basis of the interpretation of the registered signals of brain activity to the robotic device and / or to the visual display device on a transmit-receive basis.

It is possible to implement a neurorehabilitation system in which a virtual reality device is used as a visual display device.

It is possible to implement a neurorehabilitation system in which an exoskeleton is used as the robotic device, for example, an exoskeleton of a limb.

It is possible to implement a neurorehabilitation system in which an electroencephalograph (EEG) is used as a device for recording signals of brain activity.

It is possible to implement a neurorehabilitation system in which a functional near infrared spectroscopy device is used as a device for recording signals of brain activity.

It is possible to implement a neurorehabilitation system in which a magnetic resonance imaging device is used as a device for recording signals of brain activity.

It is possible to implement a neurorehabilitation system, in which a device for recording magnetic fields arising from the electrical activity of the brain is used as a device for recording signals of brain activity.

It is possible to implement a neurorehabilitation system in which at least two different registration devices are used together to register brain activity.

It is possible to implement a neurorehabilitation system, which additionally contains an electrostimulation device to stimulate the muscles and nerves responsible for moving the trained obj ect.

It is possible to implement a neurorehabilitation system, which additionally contains an electromyograph for recording the electrical activity of the muscles that set the trained object in motion, as well as for recording the activity of the corresponding muscles of the untrained object, namely the muscles of the healthy opposite limb (untrained object).

The technical result is achieved by a neurorehabilitation method using a neurorehabilitation system, including:
visual presentation by a visual display device of a task to perform a movement by a trained obj ect,
registration of brain activity signals by a brain activity recording device,
transmission of registered brain activity signals to a computer with software associated with a database,
extracting signals of brain activity necessary for the interpretation by a computer with software,
interpretation of the selected signals by comparison with a database,
transmission of a command formed on the basis of the interpretation of recorded signals of brain activity to a robotic device for impacting a trained object,
the impact of a robotic device on a trained object in accordance with the registered signals of brain activity and the command received,
transmission of a signal to a visual display device,
visual presentation of the task being performed on a visual display.

It is possible to achieve the claimed result by a neurorehabilitation method using a neurorehabilitation system, in which signals of visual evoked potential are extracted at the stage of extraction and recognition of the registered signals of cerebral activity.

It is possible to achieve the claimed result by a neurorehabilitation method using a neurorehabilitation system, in which a signal of the motor imagination is extracted at the stage of extraction and recognition of the registered signals of brain activity.

It is possible to achieve the claimed result by a neurorehabilitation method using a neurorehabilitation system, in which at the stage of extraction and recognition of registered signals of brain activity signals of visual evoked potential and motor imagination are both extracted.

It is possible to achieve the claimed result by a neurorehabilitation method using a neurorehabilitation system, in which signals of brain activity related to a healthy object are registered to impact a trained object using a robotic device.

It is possible to achieve the claimed result by a neurorehabilitation method using a neurorehabilitation system, in which, in addition to the signals of brain activity, signals of muscular activity are registered.

It is possible to achieve the claimed result by a neurorehabilitation method using a neurorehabilitation system, in which the impact on the trained object by a robotic device is performed in accordance with the muscular activity of a healthy object.

It is possible to achieve the claimed result by a neurorehabilitation method using a neurorehabilitation system, in which a signal is transmitted to a visual display device from a robotic device.

It is possible to achieve the claimed result by a neurorehabilitation method using a neurorehabilitation system, in which the impact on the trained object by a robotic device in accordance with the recognized signals of brain activity is additionally accompanied by electrical stimulation of the muscles and nerves that set the trained object in a given movement.

It is possible to achieve the claimed result by a neurorehabilitation method using a neurorehabilitation system, in which the selection and / or adjustment of the software classifier used to form the database is carried out automatically.

It is possible to achieve the claimed result by a neurorehabilitation method using a neurorehabilitation system, in which the interpretation data of brain activity obtained in the course of the task, including the data obtained during the physical impact of a robotic device on an object, is recorded into a database.

It is possible to achieve the claimed result by a neurorehabilitation method using a neurorehabilitation system, in which the degree of completion of the assigned task is displayed on the visual display device in the instant feedback mode based on the registered signals of brain activity.

It is possible to achieve the claimed result by a neurorehabilitation method using a neurorehabilitation system, in which the visual presentation on a visual display device of the task being performed is implemented in a manner that stimulates the activation of mirror neurons.

It is possible to achieve the claimed result by a neurorehabilitation method in which the assignment of the task, the registration of signals and the performing of the task are divided into several stages, and at each stage of the task, bioelectrical activity can be recorded by various devices, and actions can be performed with different parts of the trained object, while each stage is displayed on display device independently.

### Description of the illustrations

Figure 1 shows a block diagram of the interaction of the main components of the neurorehabilitation system.
Figure 2 shows a set of elements of a neurorehabilitation system.
Figure 3 shows a general view of the neurorehabilitation system (an example of implementation for the rehabilitation of the upper limbs).
Figure 4 shows a general incomplete view of the neurorehabilitation system (an example of implementation for the rehabilitation of the lower limbs).
Figure 5 shows a block diagram of the sequence of actions when implementing the method of neurorehabilitation (in the basic version).
Figure 6 shows a block diagram of the sequence of actions in the implementation of the method of neurorehabilitation, in which at the stage of extraction and recognition of the registered signals of brain activity, the signals of visual evoked potential and motor imagination are extracted.
Figure 7 shows a block diagram of the sequence of actions when implementing the method of neurorehabilitation, in which, in addition to the signals of brain activity, signals of muscular activity are registered.
Figure 8 shows a block diagram of a sequence of actions when implementing a method of neurorehabilitation, in which the impact on a trained object by a robotic device in accordance with the recognized signals of brain activity is additionally accompanied by electrical stimulation of the muscles and nerves that set the trained object in a given movement.
Figure 9 shows a block diagram of the sequence of actions when implementing the method of neurorehabilitation, in which the visual presentation on the visual display device of the task being performed is implemented in a manner that stimulates the activation of mirror neurons.
Figure 10 shows a block diagram of the sequence of actions when implementing the method of neurorehabilitation, which combines all the options for implementing the method of neurorehabilitation.

Position 1 - visual display device;
Position 2 - device for registering brain activity;
Position 3 - computer;
Position 4 - robotic device;
Position 5 - electrostimulator;
Position 6 - electromyograph;
Position 7 - motion tracker;
Position 8 - controller unit;
Position 9 - emergency movement stop button;
Position 10 - visual presentation of the task by the visual display device;
Position 11 - registration of signals of brain activity by a device for registering brain activity;
Position 12 - transmission of signals of brain activity to a computer with software and database;
Position 13 - extraction and recognition of registered signals of brain activity by a computer and interpretation by comparison with a database;
Position 14 - transmission of a command, based on the interpretation of the registered signals of brain activity, to a robotic device for impacting the trained object;
Position 15 - the impact of the robotic device on the trained object in accordance with the recognized signals of brain activity;
Position 16 - transmitting a digital signal to a visual display device;
Position 17 - extraction of the signals of the visual evoked potential;
Position 18 -extraction of the motor imagination signals;
Position 19 - registration by an electromyograph of the activity of the muscles that set the trained obj ect in a movement;
Position 20 - registration by the electromyograph of the activity of the muscles of the untrained object, corresponding to the muscles of the trained object setting it in the given movement;
Position 21 - electrical stimulation of the muscles that set the trained object in a given movement;
Position 22 - stimulation of the activation of mirror neurons.
Position 23 - the complete neurorehabilitation system as a whole.

### DETAILED DESCRIPTION

In the following detailed description of an implementation of the invention, numerous implementation details are set forth in order to provide a thorough understanding of the present invention. However, it will be obvious to those skilled in the art how the present invention can be used with or without these implementation details. In other instances, well-known methods, procedures, and components have not been described in detail so as not to obscure the details of the present invention.

Moreover, it is clear from the foregoing disclosure that the invention is not limited to the foregoing implementation. Numerous possible modifications, changes, variations and substitutions, while retaining the essence and form of the present invention, will be apparent to those skilled in the art.

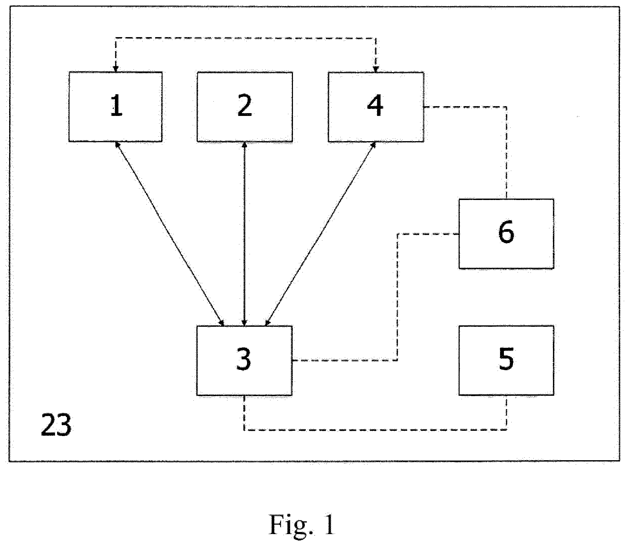

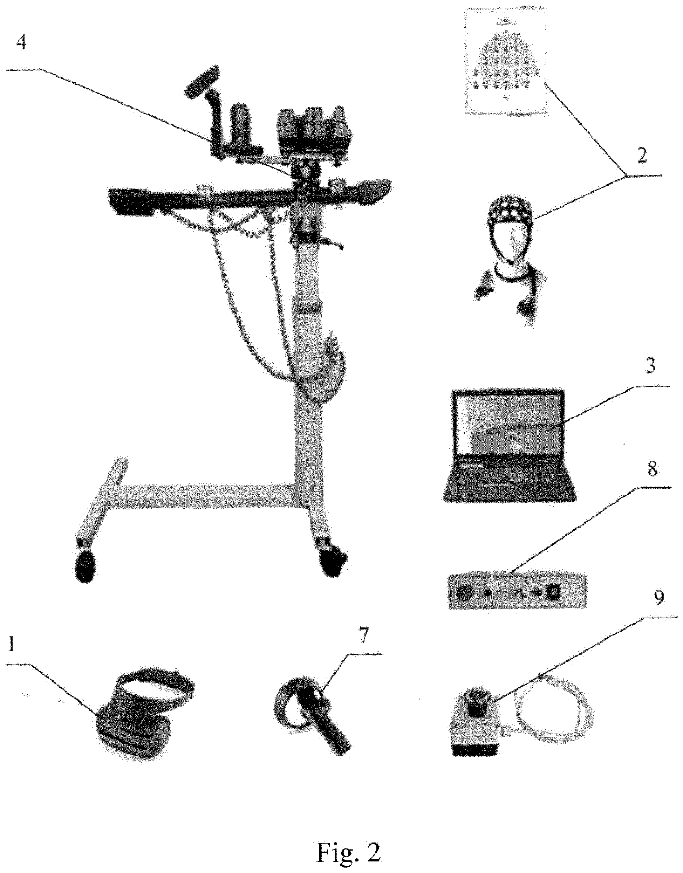

The neurorehabilitation system 23 (shown in Fig. 1 as a block diagram of the interaction of system elements) includes a visual display device 1, which, usually, is a virtual reality device, which can be represented in the form of virtual reality headset (Fig. 2) as well as a virtual reality helmet. The virtual reality helmet can be equipped with a device for playing and listening to an audio signal, which allows the patient to be more involved in an imaginary process. The use of the visual display device 1 in the neurorehabilitation system allows to visually present the task and its completion to the patient. Thus, the patient is involved in an imaginary process that makes the brain "believe" in the reality of the connection between the intention to make a movement and the real movement of the trained object, i.e. paralyzed limb, which contributes to an increase in the effectiveness of motor rehabilitation, including in stroke and in other diseases and disorders of the central nervous system. In addition, the visual display device 1, including in the embodiment in the form of a virtual reality device, makes it possible to visually display an animated example of a task execution - for example, in the form of a virtual phantom, "performing" specific movements, while in reality a trained object (for example, the arm) is at rest. Observing a moving phantom facilitates the mental task of motor imagination. It is also possible to display the progress of the task "from the third person view" that is, the patient is presented with a view of themselves "from aside" during the movement of the limb. Observing the task from the third person stimulates the activation of mirror neurons, which facilitates the restoration of neural connections. Thus, these modes of displaying the task and the course of its fulfillment increase the efficiency of motor rehabilitation.

The neurorehabilitation system 23 (Fig. 1) also includes a device for recording brain activity 2, which registers and transmits signals of brain activity to the computer 3, forming a brain-computer interface, the task of which is to register, process, extract and interpret brain activity in order to determine the patient's intention to move the trained (or untrained) object. An electroencephalograph (Fig. 2) or a similar device can be used as a device for recording brain activity 2 that records the electrical and bioelectric activity of the brain from the surface of the scalp. A near-infrared spectroscopy (NIRS) device can also be used to measure brain activity through hemodynamic responses associated with neuroactivity; also, a magnetic resonance imaging (MRI) device can be used that records nuclear magnetic resonance signals, as well as devices that read and register magnetic fields arising from the electrical activity of the brain. The most preferred in practice options for use as a device for recording brain activity 2 are an electroencephalograph and a near infrared spectroscopy device. In addition, the listed devices can be used in various combinations; the most preferable variant of the combination of devices for recording brain activity 2 is the combined use of an electroencephalograph and a near infrared spectroscopy device. The combined use of an electroencephalograph and a near-infrared spectroscopy device makes it possible to increase the accuracy of detecting and registering signals of brain activity and to better recognize the patient's intentions. Therefore, it has a positive effect on improving the effectiveness of rehabilitation. A combination of these technologies can be implemented in a single compact device.

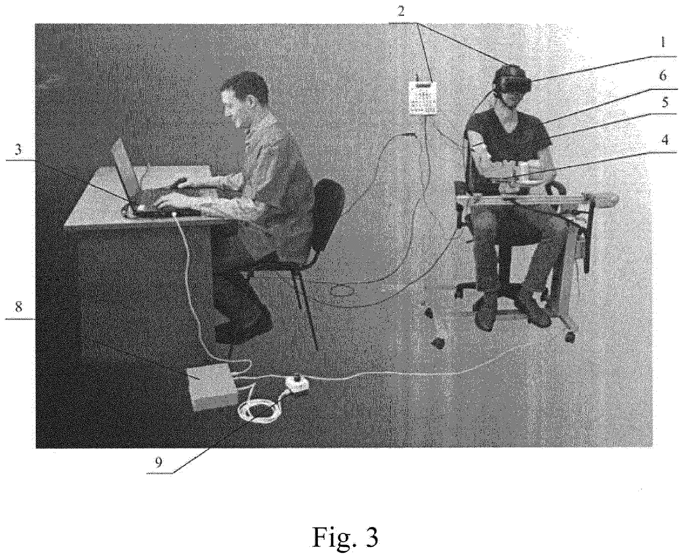

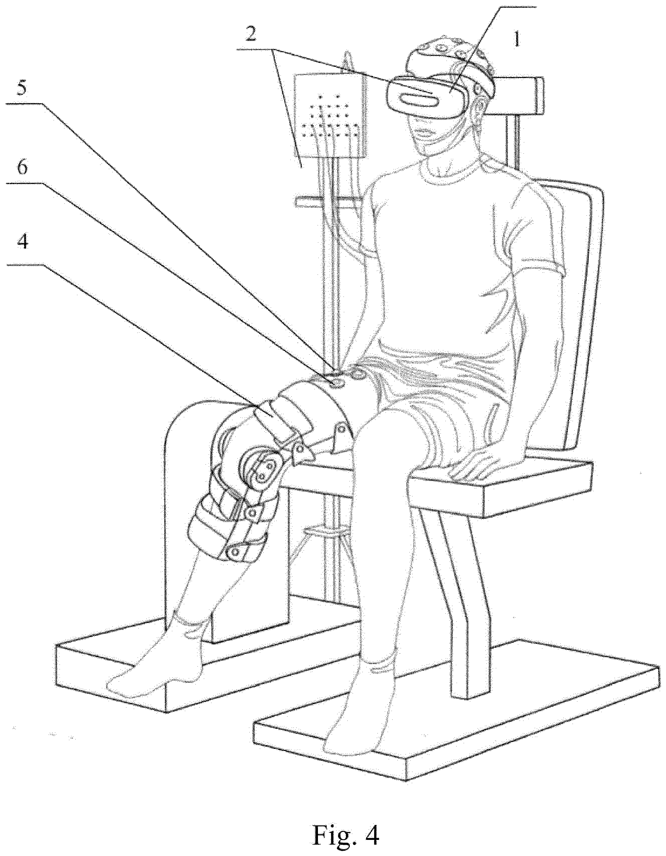

The neurorehabilitation system 23 (Fig. 1) also includes a robotic device 4, which is used for physical interaction with a trained object, that is, a paralyzed, paretic, rehabilitated limb, including for moving the trained object in accordance with the recognized signals of brain and / or muscle activity. Also different variants of the implementation of the robotic device 4, including an exoskeleton, are possible; they are intended for training and rehabilitation of the upper (Fig.3), lower (Fig. 4) limbs, as well as individual parts of the limbs. In particular an exoskeleton of a limb (Fig. 4) can be used as a robotic device. In this case, the robotic device 4 can be configured to transmit a digital signal to the visual display device 1 directly, as well as through the computer 3. The biofeedback that arises as a result of the analysis of neurophysiological signals and the corresponding complex cognitive, proprioceptive and kinesthetic effects on the patient's body stimulates neuroplasticity and thus has a positive effect on the effectiveness of rehabilitation.

The neurorehabilitation system 23 (Fig. 1) also includes a computer 3, which contains software for recognizing and extracting the registered signal and interpreting it using a database that can be located both on the computer 3 and on a separate device, as well as on server or cloud storage. In the neurorehabilitation system 23 (Fig. 1), the computer 3 transmits the commands formed on the basis of the interpretation of the registered signals of brain and / or muscle activity to the robotic device 4 and / or to the visual display device 1 according to the transmit-receive principle. Computer 3 (Fig. 1) in combination with a device for recording brain activity 2 form a brain-computer interface. The signals of brain activity are sent from the brain activity registering device 2 to the computer 3, where, using the software installed on it, patterns are recognized and identified in the signals, allowing determining the activity that the patient intends to execute by performing a limb movement, that is, to determine the chosen goal or movement. At the same time, computer 3 with software interprets the signals of brain activity using software classifiers that compare the incoming signals with the reference patterns of brain activity in the database. Interpretation of signals of brain activity using software classifiers can be implemented using various mathematical techniques, including the technology of artificial neural networks, by identifying characteristic features (patterns) of brain activity, for example, associated with external stimulation or cognitive activity, and then searching for similar patterns in recognized, extracted, interpreted signal of brain activity. At the same time, the classifiers are adaptively reconfigured, that is, they are "trained", both automatically and manually, adjusting to specific tasks and a specific patient. Automatic and manual selection of classifiers is possible, which makes it possible to increase the accuracy of their work and reduce the training time of the classifier. Computer 3, on which the program classifiers are located, makes it possible to extract and recognize both patterns of motor imagination and patterns of visual evoked potentials (an electric wave subconsciously arising in the cerebral cortex as a reaction to a "significant" visual stimulus: for example, to a change in brightness - "flashing" of the object on the visual display device 1, on which the patient has focused their attention). Due to the "subconscious" appearance and characteristic features of the pattern of the visual evoked potential, which allow it to be detected with high accuracy in the signals of brain activity, this paradigm requires less mental effort from the patient and thus can be applied to patients with a reduced cognitive ability typically occurring in early stroke. In this case, it is possible to extract, recognize and further work with both the signal of the motor imagination and the signal of the visual evoked potential, as well as combined extraction, recognition and further work with these two types of signals. Thus, the use of a computer 3 with software for recognizing and extracting a registered signal and interpreting the extracted registered signal using the database that can be located on a computer, contributes to an increase in the effectiveness of rehabilitation.

It should be noted that computer 3, on which the software is installed, can be located in a personal computer; this is-the most preferable option. Computer 3 can also be located in a mobile device, for example a smartphone. Additionally, it is possible to locate computer 3 remotely, for example, on a server, on a device in a local network or in the cloud. It is also possible to arrange the computer 3 on a microcomputer or several microcomputers built into one or more of the system elements according to claim 1, for example, into a visual display device 1 or into a robotic device 4, etc.

It is possible to implement a neurorehabilitation system 23 (Figure 1), which additionally contains an electrostimulator 5 to stimulate the muscles and nerves that set the trained object in a given movement. The electrostimulator 5 makes it possible to implement electrical stimulation in the neurorehabilitation system, which consists of the simultaneous movement of the trained object by the robotic device 4 with a specific electrical effect through the skin on certain muscle and nerve fibers in order to enhance biofeedback and stimulate the muscles own activity and the formation of patterns of neuronal activity corresponding to the implementation of desired movements. In addition, when using electrical stimulation, the work of the locomotor centers is normalized at all vertical levels of motor activity regulation, and the maximum restructuring of the patient's neural dynamics is achieved. Thus, the additional use of the electrostimulator 5 in the neurorehabilitation system increases its efficiency.

It is possible to implement a neurorehabilitation system 23 (fig. 1), which additionally contains an electromyograph 6 for registering muscular activity that sets a trained object in motion. Electromyograph 6, with sensors placed on the skin over certain muscles, is designed to register bioelectric potentials of muscular activity and thus allows to register muscle activity, that is, to register and measure electrical and physical tension in the muscles. This way, the patient's muscle tension can be registered and recorded. If the activity of the muscles driving the trained object is strong enough, an additional condition for the start of movement can be the performance of a certain effort by the patient's muscles, measured by the electromyograph 6 with sensors placed on the muscles that set the trained object in motion, and the patient, in addition to imagination of movement, should try to perform this movement. A variant is possible when the movement of the trained object by the robotic device 4 in accordance with the recognized signals of brain activity occurs under the condition of a sufficient level of the activity of the muscles of the untrained object, corresponding to the muscles setting the trained object in the given movement, recorded by the electromyograph, that is, when the muscle tension of the healthy limb sets in motion the affected limb. A gradual increase in the level of one's own muscular effort required to activate the robotic device helps to restore the limb's own motor activity. Thus, the use of electromyograph 6 in the neurorehabilitation system increases the effectiveness of rehabilitation.

To understand the work and functioning of the neurorehabilitation system 23 shown in **Figure 1****,** below is an example of the implementation of its work and the functioning of its elements. This example is given in order to provide an opportunity for a person skilled in the art to understand the principles of interaction of system elements and the principles of operation and functioning of the system 23 as a whole and should be considered as illustrative and not limiting the scope of the invention:

Using a visual display device, tasks are given to the patient related to the execution of movements by a paralyzed (trained) limb. In the course of performing the task, the patient must imagine the movement of the limb to the selected target, to the selected position or in the selected direction. At the same time, the brain activity registration device 2 registers the signals of brain activity. For example, it can be an electroencephalogram (EEG) of a patient, based on which a software classifier identifies patterns of electrical activity in the brain, allowing to determine the selected target or movement. Thereafter, a command is issued to the robotic device 4 (for example, an exoskeleton) to act on the limb (for example, to move it) in accordance with the detected intention of the patient.

If the patient's own muscle activity, which sets the rehabilitated limb in motion, is strong enough, an additional condition for starting movement can be the execution of a certain self-effort by the patient's muscles. In this case, an electromyograph 6 is additionally used, with sensors placed on the muscles that set the limb in motion, and the patient, in addition to imagining movement, should try to perform such a movement. An option is also possible when the tension of the muscles of a healthy limb sets the affected limb in motion.

The above description concerns and is based mainly on Fig. 1, which shows a block diagram of the interaction of the elements of the neurorehabilitation system. For clarity of the implementation of the system, a description of Figures 2-4 is also provided. In this case, the above embodiments are to be considered in all respects only as illustrative and not limiting the scope of the invention.

**Figure 2** shows a variant of a set of elements of a neurorehabilitation system, which includes:
a visual display device 1, presented in the form of virtual reality headset, provides a visual presentation 10 of a task for performing a movement by a trained object and contributes to a clearer and more imaginative presentation by the patient of the task being performed and, accordingly, amplification of brain activity to complete the task, thereby increasing the efficiency of the formation of signals of brain activity;
a device for recording brain activity 2, presented in the form of an electroencephalograph, capable of registering bioelectric activity of the brain from the surface of the scalp using sensors placed directly on the patient's head;
computer 3, presented in the form of a laptop, which contains software for recognizing and extracting the registered signal and interpreting the extracted registered signal using a database. The computer 3 transmits the commands formed on the basis of the interpretation of the registered signals of brain activity to the robotic device 4 and to the visual display device 1 according to the transmit-receive principle;
robotic device 4, presented in the form of one of the variants of the upper limb exoskeleton, serves for physical interaction with the trained object (that is a paralyzed, paretic, rehabilitated limb), including for moving the trained object in accordance with the recognized signals of brain and / or muscle activity;
motion tracker 7, which can be used during the operation and functioning of the robotic device 4 and allows to determine the position of the trained object in space, which is then transmitted to the visual display device 1, for more accurate and realistic visualization of the task being performed;
a controller unit 8, which controls the operation of the drives of the robotic device 4, and is not an obligatory element of the system and is given only as an example of implementation. For example, nowadays such versions of exoskeletons or other robotic devices are being produced, that do not need a dedicated controller unit for their operation, or have a built-in controller unit;
an emergency stop button 9 that is intended for an emergency stop of the robotic device 4 in case of an emergency situation, and can be external and built-in. Thus, the emergency stop button 9 contributes to an increase in the safety of operation of the neurorehabilitation system 23. It is possible to use other tools to ensure the safety of the operation of the system.

Figure 3 depicts a perspective view of the neurorehabilitation system in action (for example of upper limb rehabilitation), which comprises:
a visual display device 1;
a device for recording brain activity 2;
computer 3;
robotic device 4;
electrostimulator 5 (Fig. 3 shows the electrode of the electrostimulator 5) for stimulating the muscles and nerves that set the object to be trained in a given movement. The electrostimulator 5 makes it possible to implement electrostimulation in the neurorehabilitation system, which consists in the simultaneous movement of the trained object by the robotic device 4 and electrical action on the corresponding muscles and nerves in order to enhance biofeedback, stimulate the own muscular activity and the formation of patterns of neuronal activity corresponding to the implementation of target movements. In addition, when using electrical stimulation, the work of the locomotor centers is normalized at all vertical levels of regulation of motor activity, and the maximum restructuring of the patient's neurodynamics is achieved;
electromyograph 6 (Figure 3 shows the electrode of electromyograph 6) thet provides registration of the activity of the muscles which set the object in motion. Electromyograph 6 with sensors is designed to register bioelectric potentials of muscle activity and, indirectly, measure the physical effort in them. Thus, the level of the patient's own muscular effort can be determined. Accordingly, one of the conditions for the movement of the trained object by the robotic device 4 in accordance with the recognized signals of brain activity may be the patient's tension of his own muscles up to a certain level, measured by the electromyograph 6;
controller unit 8;
emergency movement stop button 9;

Figure 4 depicts a partial view of the overall system neurorehabilitation (an example of implementation for the rehabilitation of lower extremities), comprising:
a visual display device 1;
a device for registering brain activity 2;
robotic device 4, presented in the form of one of the variants of the lower limb exoskeleton;
electrostimulator 5 (Figure 4 shows the electrode of the electrostimulator 5). In this example, the electrostimulator electrode 5 is located on the extensor muscle of the leg (quadriceps femoris) in the area of the knee joint, and stimulates its work;
electromyograph 6 (Figure 4 shows the electrode of the electromyograph 6). In this case, the electromyograph sensor 6 is located in the region of the patient's quadriceps femoris (quadriceps), which serves to extend the leg at the knee joint.

By using the elements described above, the claimed invention "brain-computer interface-based neurorehabilitation system" increases the effectiveness of rehabilitation after a stroke and other diseases and disorders of the central nervous system by stimulating the restoration of mobility of the paralyzed limb by formation of neural biological feedback between the patient's intention to make a movement and its implementation. The neurorehabilitation system 23 (Fig. 1) allows registering, recognizing and extracting signals of brain and muscular activity, identifying the patient's intention to make a movement with a trained paralyzed object or its healthy analog, and then helping to perform this movement. The resulting biofeedback stimulates neuroplasticity - a process in the brain that forms bypass neural pathways to replace those lost or damaged as a result of the disease. Additionally, the effectiveness of rehabilitation is increased through the use of a visual display device. Thus, the problem of neurorehabilitation of patients with post-stroke neural symptomatic is being solved.

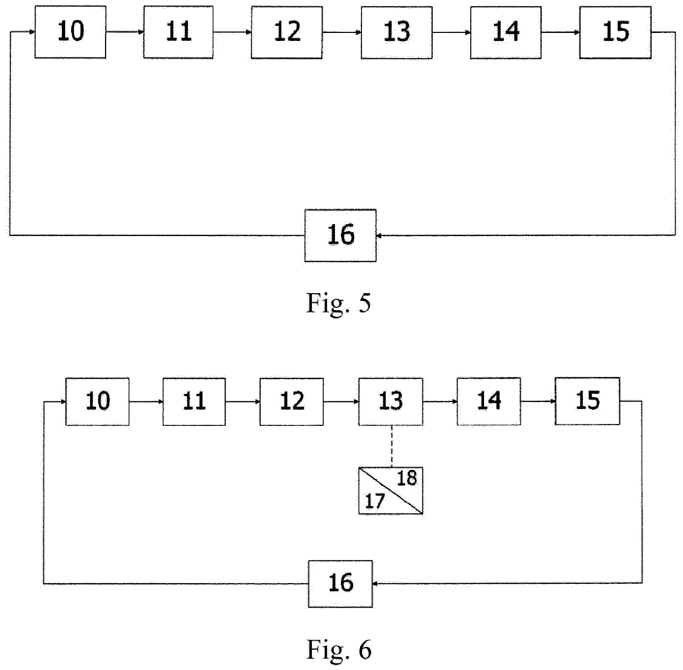

The method of neurorehabilitation using a neurorehabilitation system (in the basic version) is characterized by at least the following sequential actions, namely (see Fig. 5):
10 - visual presentation of a task by a visual display device;
11 - registration of signals of brain activity by a device for recording brain activity;
12 - transmission of signals of brain activity to a computer with software associated with the database;
13 - extraction of signals of brain activity necessary for interpretation by a computer and their interpretation by comparing them with a database;
14 - transmission of a command, formed on the basis of interpretation of registered signals of brain activity, to a robotic device for impacting a trained object;

- the impact of a robotic device on a trained object in accordance with the recognized signals of brain activity;
- transmission of a digital control signal to a visual display device.

Neurorehabilitation using a neurorehabilitation system is carried out according to the following **method:**
the patient is given tasks in visual and / or auditory form related to the execution of movements of a paralyzed limb. The visual presentation of the task can take place in a virtual reality environment using a virtual reality device that can be equipped with means of playing an audio signal. This allows the patient to be more involved in an imaginary process with the help of additional sound stimulation. At the same time, audio feedback can be implemented to display the quality of the task performance - for example, using signals of different volume and tone; or in the case of performing the assigned task with high quality, a melodic sound may play. The visual presentation of the task 10 in the virtual reality environment increases the manifestations of brain activity when performing the task, thereby positively affecting the effectiveness of rehabilitation;
furthermore, in the course of performing the task, the patient imagines the fulfillment of the given task of moving the trained object, that is, imagines the movement of the limb to the selected target, to the selected position or in the selected direction, thereby performing the given brain activity, and the brain activity registering device 2 registers and transmits signals of brain activity to the computer 3;
registration and subsequent transmission to the computer 3 of signals of brain activity is possible using an electroencephalograph as a device for recording brain activity 2;
it is possible to register and transmit to a computer 3 signals of brain activity using near-infrared spectroscopy devices (NIRS - near-infrared spectroscopy) measuring hemodynamic reactions associated with neural activity as a device for recording brain activity 2 ;
it is possible to register and transfer to the computer 3 information about brain activity in the form of information about the level of induced nuclear magnetic resonance in brain cells registered by a magnetic resonance imaging (MRI) device;
it is possible to register and then transmit to the computer 3 information about brain activity by registering the level of magnetic fields arising from the electrical activity of the brain, a magnetic encephalography (MEG) device or the like.

In the proposed method, the most preferred option is the registration and subsequent transmission 12 to the computer 3 of signals of electrical and bioelectric activity of the brain using an electroencephalograph as a device for registering brain activity 2. Another preferred option is the registration and subsequent transmission 12 to the computer 3 of signals of brain activity by using a near infrared spectroscopy device.

Also, various combinations of the above options for recording bioelectric signals are possible; the most preferred combination is the combined use of registration and subsequent transmission 12 to the computer 3 of signals of electrical and bioelectric activity of the brain using an electroencephalograph as a device for registering brain activity 2, together with the registration and subsequent transmission 12 to the computer 3 of NIRS signals. The combination of options allows to increase the accuracy of registration of signals of brain activity, and thus allows better interpretation and an increase in the effectiveness of rehabilitation;

The next step is registration and recognition of the registered signals of brain activity and their interpretation 13 by the computer 3 by comparing them with the database to identify patterns of brain activity, allowing to determine the selected target or movement. The registered signals of brain activity are sent from the brain activity recording device 2 to the computer 3. Using the software installed on the computer 3 (which can also be located on a server, on a third-party device or in a cloud storage, and calculations are performed by distributed computing by several devices located remotely on a server, on a third-party device, or in a cloud storage), the signals are recognized and interpreted. These signals reveal patterns of brain activity that allow the software to determine the chosen target or movement. At the same time, computer 4 with software interprets the signals of brain activity using software classifiers and a database containing reference signals of brain activity. Interpretation of signals of brain activity with the help of software classifiers can be implemented on the basis of artificial neural network technologies that use various mathematical methods to identify specific features (patterns) of brain activity associated with external stimulation or cognitive activity and then search for such patterns in the interpreted signal. At the same time, the results of the interpretation of brain activity are recorded in the database for its expansion. In addition, classifiers are adaptively reconfigurable, that is, they are "trained", adjusting to specific tasks and a specific patient. Automatic and manual selection of the optimal classifiers and their parameters is possible, which improves the accuracy and speed of their work and reduces the training time. Computer 3 (on which the software for recognition and extraction of the registered signal and interpretation of the extracted registered signal using the database, can be located), makes it possible to extract and recognize the signals of the visual evoked potential 17 and the signals of the motor imagination 18 (Fig. 6). Motor imagery signals 18 are usually synchronization / desynchronization (i.e., increase / suppression) of various rhythms of brain activity in the area of limb representation in the motor cortex and other parts of the cortex. The visual evoked potential signal 17 (Fig. 6) is an electrical wave that unconsciously arises in the cerebral cortex as a reaction to a "significant stimulus" - for example, to visual highlight (or change in brightness - "backlight") of an object on which the patient focuses their attention. Due to the "unconscious" nature of the occurrence and the characteristic features of the patterns of visual evoked potential, which allow them to be identified with high accuracy in signals of brain activity, the use of the paradigm for the extraction of visual evoked potentials 17 requires less mental effort from the patient and can be applied for patients with reduced cognitive level that can occur in early stroke. In this case, it is possible to extract, recognize and further work separately with the signal of the motor imagination 18 and the signal 17 of the visual evoked potential. Their combined extraction, recognition and further work with these signals is also possible. Additionaly, to facilitate the training of motor imagination for the patient and to facilitate the training of the classifier, kinesthetic training can be used when the trained object is moved by the robotic device 4, and the patient is instructed to imagine the corresponding muscle activity during the movement of the limb. Thus, the extraction and recognition of (registered) signals of brain activity and interpretation 13 by the computer 3 by comparing it with the database contributes to the increase in the effectiveness of rehabilitation.

After the extraction and recognition of the registered signals of brain activity and interpretation 13 by the computer 3 by comparison with the database, the command 14 is transmitted to the robotic device to impact the trained object 15, for example, to perform the intended movement. In this case, an exoskeleton can be used as a robotic device 4, which allows optimal anatomical parameterization. Thus, the transmission of the command 14, formed on the basis of the interpretation of the registered signals of brain activity, to the robotic device, and the subsequent action of the robotic device on the trained object 15 stimulate the formation of new neural connections in the brain instead of those lost, using the formation of neural biofeedback between intention and movement. Thus, the use of a robotic device in the system increases the effectiveness of rehabilitation.

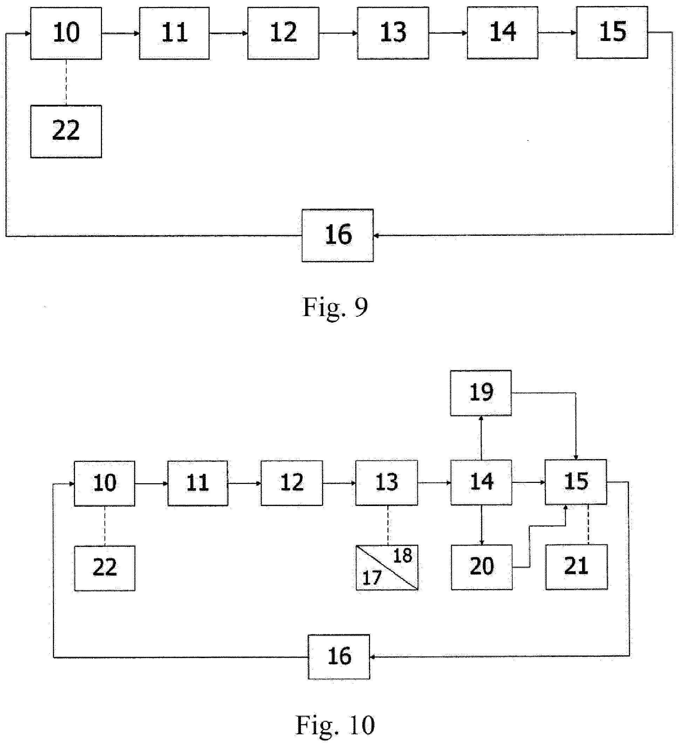

During the action of the robotic device, the patient observes the task execution process on the visual display device 1: signal 16 is transmitted to the visual display device 1 and then visual presentation 10 takes place on the visual display device 1 of the task execution process, including that in accordance with the signal received from the robotic devices. Thus, the patient is involved in an imaginary process that makes the brain "believe" in the reality of the connection between the intention to make a movement and the physical movement of the trained object, i.e. paralyzed limb, which improves the effectiveness of rehabilitation. In addition, the visual display device 1, including that implemented in the form of a virtual reality device, provides a visual display of the performance of a task "from a third person", that is, to show the patient a view of themselves "from the side", or to display a virtual phantom "performing" given movement, while the really trained object, for example, an arm, is at rest. Observing a moving phantom (or observing the execution of a task "from the third person") facilitates the performance of a mental task and stimulates the activation of mirror neurons 22 (Fig. 9), facilitating the restoration of neural connections, which also increases the efficiency of rehabilitation.

It is possible to implement the neurorehabilitation method, in which the command to impact 15 the trained object is sent from the computer 3 to the robotic device 4, and after the impact of the robotic device 4 on the object - to the visual display device 1. In this case, the command can be transmitted both directly from the robotic device 4 to the visual display device 1 and via the computer 3. Such an embodiment is necessary, for example, when a movement tracker 7 is used in the robotic device 4, which determines the position of the trained object in space. Information about the spatial position of the trained object is transmitted to the visual display device 1 for a more accurate and realistic visualization of the task being performed. At the same time, it is possible to transfer a digital signal from the robotic device 4 to the visual display device 1 through the computer 3 for its preliminary processing, transformation and conversion into the desired format perceived by the visual display device 1.

It is possible to implement the neurorehabilitation method, in which the impact on the trained object by the robotic device 4 is produced on the basis of recognized brain activity signals related to a healthy object. This option is particularly applicable in cases of severe damage to motor ability in early stroke, when it is difficult for the patient to perform controlled brain activity in relation to the trained object (affected limb). That is, if patients have difficulty in performing the task of motor imagination with the affected hemisphere of the brain, it is advisable to perform the exercise using both limbs (e.g. arms), when they have the opportunity to periodically perform the task for the healthy arm and then try to reproduce it with the paralyzed limb "by analogy", which increases the effectiveness of rehabilitation.

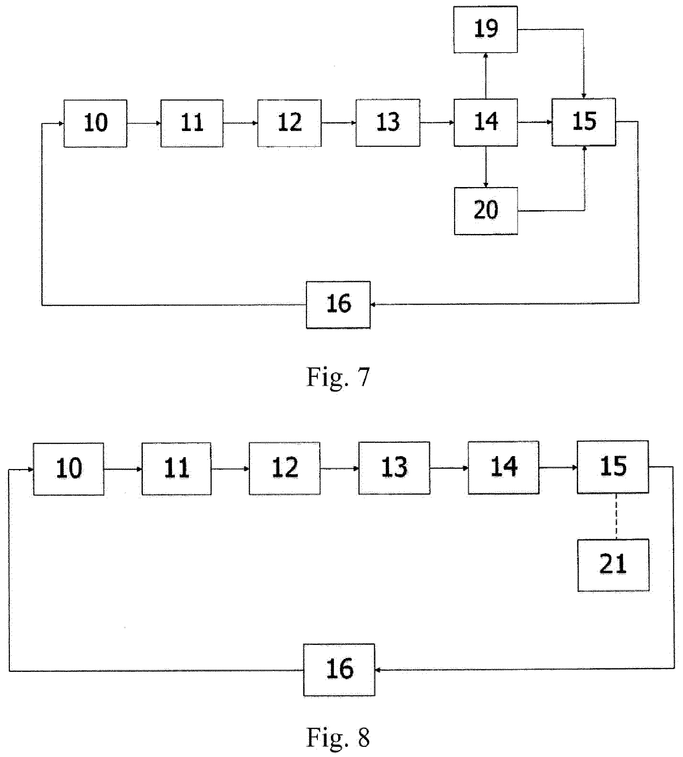

It is possible to implement the method of neurorehabilitation (Fig. 7) in which the impact of the robotic device 4 on the trained object in accordance with the recognized signals of brain activity 15 occurs under the condition of the activity of the muscles that set the trained object in a given movement, registered by the electromyograph, that is, after the selection and recognition (registered) signals of brain activity by a computer and interpretation 13 by comparing it with a database, the electromyograph additionally records the activity of the muscles that set the trained object 19 in a given movement, and provided that the patient's own muscles are contracted to a certain level, measured by the electromyograph 6, the trained object is moved by a robotic device in accordance with the recognized signals of brain activity 15. In addition, it is possible to implement a method in which the movement 15 of a trained object, that is a paralyzed limb, by a robotic device 4 in accordance with the recognized signals of brain activity occurs under the condition of sufficient activity of the muscles of the corresponding untrained object registered by the electromyograph, that is a healthy limb, corresponding to the training object setting in a given movement, i.e. when muscle tension in a healthy limb sets the affected limb in motion. Later, the patient "by analogy" tries to induce muscle activity of the affected limb. Thus, the registration of the activity of muscles 19 by the electromyograph and the movement 15 of the trained object by the robotic device 4, provided that the patient's own muscles are contracted to a certain level, measured by the electromyograph 6, increases the efficiency of rehabilitation.

It is possible to implement the neurorehabilitation method (Fig. 8), in which the movement 15 of the trained object by the robotic device 4 in accordance with the recognized signals of brain activity is accompanied by additional electrical stimulation 21 of muscles and nerves that set the trained object in a given movement. Electrostimulation 21 is performed using an electrostimulator 5. In response to the performance of a mental and / or muscular task, a complex stimulation of the motor system occurs, which consists in simultaneous functional electrical stimulation of the corresponding muscles and nerves when the 15 trained object is moved by a robotic device 4 in order to enhance biofeedback and stimulate its own muscle activity and the formation of patterns of neuronal activity corresponding to the performance of targeted movements. In addition, when using electrical stimulation, the work of the locomotor centers is normalized at all vertical levels of regulation of motor activity, and the maximum restructuring of the patient's neurodynamics is achieved. Thus, the use of electrical stimulation 21 contributes to an increase in the effectiveness of rehabilitation.

It is possible to implement the neurorehabilitation method, in which the degree of completion of the assigned task based on the registered signals of brain activity is displayed on the visual display device 1 in the instant feedback mode, that is, when performing the task, using the visual display device 1, the patient is provided with information about how successfully they perform the task. Information can be provided in the form of a changing scale or indicator, where, if the task is performed correctly, the maximum value is displayed. If the task is performed incorrectly or not close enough to the set value, then the value displayed on the scale or indicator decreases. In addition, instant feedback on the degree of completion of the assigned task can be performed using audial signals, for example, when a virtual reality helmet with built-in devices for listening to an audio signal is used as a visual display device 1. Such an implementation allows the patient to determine and understand how correctly and efficiently they perform the task, thereby stimulating them to manifest the expected brain activity and increasing the effectiveness of rehabilitation. In addition, the patient's ability to see that when the task is performed correctly contributes to the production of neurotransmitters in the patient's body, which contribute to the restoration of neural connections in the brain.

It is possible to implement the neurorehabilitation method, in which the task, registration of signals and commands to perform the task are divided into several stages, while each stage of the task can be recorded by a different device, performed by different parts of the trained object, and each stage is displayed on the visual display device 1 independently. Thus, the invention makes it possible to simulate the implementation of complex multi-stage movements, similar to those performed by the patient in real life, and thus to carry out complex rehabilitation of the limb. In this case a more and less intact function are immediately restored in the same exercise. For example, the mobility of the entire arm as a whole can be restored based on the analysis of the electromyogram of the large muscles of the shoulder, and the mobility of the hand can be restored based on the analysis of the electroencephalogram. An example is the "reach out and take a glass" task. The patient must first tense the shoulder muscles so that the robotic device moves the entire arm in the direction of the virtual glass, and then imagine the contraction of the forearm muscles so that the robotic device physically closes their hand, helping them to "take" the glass.

In addition, all of the above options for implementing the method of neurorehabilitation can be combined: all together (Fig. 10) or in various combinations. They also can be used separately from each other.

Thus, the use of the claimed invention "neurorehabilitation system" and the claimed invention "neurorehabilitation method" increase the effectiveness of motor rehabilitation after a stroke, including in early and late rehabilitation period, and in other diseases and disorders of the central nervous system by stimulating the restoration of mobility of the paralyzed limb by formation in various ways and their combinations of neurobiological feedback between the patient's intention to make a movement and its implementation. The neurorehabilitation system allows registering, recognizing and extracting signals of brain activity, revealing the patient's intention to make a movement with the trained object, helping them to make this movement and immersing them in a virtual environment similar to ordinary life through the use of a visual display device, which also can be a virtual reality device. Thus the resulting biofeedback stimulates neuroplasticity - a process that forms bypass neural pathways to replace those lost or damaged as a result of the disease. An additional increase in the effectiveness of rehabilitation occurs with the use of electrical stimulation and electromyography.

In the present application materials, the preferred disclosure of the implementation of the claimed technical solution is presented. This should not be used as limiting other, particular embodiments of its implementation, which do not go beyond the scope of the claimed scope of legal protection and are obvious to specialists in the relevant field of technology.

## Claims

1. A neurorehabilitation system including:
a visual display device,
a device for recording brain activity,
a robotic device for impacting a trained object,
a computer with a database and software for recognizing and extracting a registered signal of brain activity and interpreting the extracted recorded signal using a database,
whereby a computer with the software is configured with the ability to transmit commands formed on the basis of the interpretation of the registered signals of brain activity to the robotic device and/or to the visual display device on a transmit-receive basis.

2. The neurorehabilitation system according to claim 1, wherein a virtual reality device is used as a visual display device.

3. The neurorehabilitation system of claim 1, wherein an exoskeleton is used as the robotic device.

4. The neurorehabilitation system according to claim 1, wherein an electroencephalograph is used as a brain activity registration device.

5. The neurorehabilitation system according to claim 1, wherein a near-range infrared spectroscopy device is used as the brain activity registration device.

6. The neurorehabilitation system according to claim 1, wherein a magnetic resonance imaging device is used as a brain activity registration device.

7. The neurorehabilitation system according to claim 1, in which a device for recording magnetic fields resulting from electrical activity of the brain is used as a device for registering brain activity.

8. The neurorehabilitation system according to claim 1, in which at least two different recording devices are used together as a device for recording signals of brain activity.

9. The neurorehabilitation system according to claim 1, further comprising an electrical stimulator.

10. The neurorehabilitation system according to claim 1, further comprising an electromyograph.

11. A method of neurorehabilitation, including:
visual presentation by a visual display device of a task to perform a movement by a trained object,
registration of signals of brain activity by a device for recording brain activity,
transmission of recorded signals of brain activity to a computer with software associated with a database,
extraction of signals necessary for interpretation of brain activity by a computer with software,
interpretation of the selected signals by comparison with a database,
transmission of commands formed on the basis of the interpretation of recorded signals of brain activity to a robotic device and / or a visual display device,
impact of a robotic device on a trained object,
signal transmission to a visual display device,
visual presentation on a visual display device the task being performed.

12. The method of neurorehabilitation according to claim 11, in which at the stage of extraction and recognition of the registered signals of brain activity, a signal of the visual evoked potential is extracted.

13. The method of neurorehabilitation according to claim 11, in which at the stage of extraction and recognition of the registered signals of brain activity, a signal of the motor imagination is extracted.

14. The method of neurorehabilitation according to claim 11, in which at the stage of extraction and recognition of the registered signals of brain activity, signals of visual evoked potential and motor imagination are extracted.

15. The method of neurorehabilitation according to claim 11, wherein the signals of brain activity related to a healthy object are registered to impact the trained object by the robotic device.

16. The method of neurorehabilitation according to claim 11, wherein, in addition to the signals of brain activity, signals of muscular activity are registered.

17. The method of neurorehabilitation according to claim 16, in which the impact on the trained object by the robotic device is performed in accordance with the muscular activity of the healthy object.

18. The method of neurorehabilitation according to claim 11, wherein the command to the visual display device is transmitted from the computer via the robotic device.

19. The method of neurorehabilitation according to claim 11, in which the impact on the trained object by a robotic device in accordance with the recognized signals of brain activity is additionally accompanied by electrical stimulation of the muscles and nerves responsible for moving the trained object.

20. The method of neurorehabilitation according to claim 11, in which the selection and / or adjustment of the software classifier used to form the database is carried out automatically.

21. The method of neurorehabilitation according to claim 11, in which the data on the interpretation of brain activity obtained during the execution of the task, including the data obtained during the physical impact of the robotic device on the object, is recorded in the database.

22. The method of neurorehabilitation according to claim 11, in which on the visual display device is being demonstrated the instant visual feedback on the degree of fulfillment of the assigned task based on the registered signals of brain activity displayed.

23. The method of neurorehabilitation according to claim 11, wherein the visual presentation on the visual display device of the task being performed is implemented in a manner that stimulates activation of mirror neurons.

24. The method of neurorehabilitation according to claim 11, in which the presentation of the task, the registration of signals and the performance of the task are divided into several stages, while at each stage of the task, the bioelectric activity can be registered by different devices, and the actions can be performed with different parts of the trained object, and each stage is displayed on the display device independently.
